# EUROPEAN PATENT APPLICATION

(11) **EP 1 538 164 A1**
(43) Date of publication of application: **08.06.2005**
(21) Application number: 03027943.4
(22) Date of filing: 04.12.2003
(51) Int. Cl.: C07K 14/78, C07K 7/06, A61K 47/48

(54) **RGD targeting moiety its production and use**

(71) Applicant: Vectron Therapeutics AG, 35037 Marburg (DE)
(72) Inventor: Kontermann, Roland, Dr., 35085 Ebsdorfergrund (DE); Höllig, Peter, 63607 Wächtersbach (DE)
(74) Representative: Zwicker, Jörk

(57) **Abstract**

The present invention relates to novel RGD comprising polypeptides, compositions and complexes comprising them as well as their use.

SEQUENCE LISTING

<110>  vectron therapeutics AG

<120>  RGD comprising peptides and uses thereof

<130>  FB12853

<160>  15

<170>  PatentIn version 3.2

<210>  1
<211>  11
<212>  PRT
<213>  Artificial

<220>
<223>  Artificial integrin binding RGD comprising peptide

<400>  1

Val Cys Gln Cys Arg Gly Asp Cys Phe Cys Pro
1               5                   10

<210>  2
<211>  11
<212>  PRT
<213>  Artificial

<220>
<223>  Artificial integrin binding RGD comprising peptide

<400>  2

Val Cys Gln Cys Arg Gly Asp Cys Leu Cys Val
1               5                   10

<210>  3
<211>  11
<212>  PRT
<213>  Artificial

<220>
<223>  Artificial integrin binding RGD comprising peptide

<400>  3

Pro Cys His Cys Arg Gly Asp Cys Leu Cys Gln
1               5                   10

<210>  4
<211>  11
<212>  PRT
<213>  Artificial

<220>
<223>  Artificial integrin binding RGD comprising peptide

<400>  4

Phe Cys Glu Cys Arg Gly Asp Cys Leu Cys Phe
1               5                   10


<210>  5
<211>  11
<212>  PRT
<213>  Artificial

<220>
<223>  Artificial integrin binding RGD comprising peptide

<400>  5

Glu Cys Gln Cys Arg Gly Asp Cys Leu Cys Pro
1               5                   10


<210>  6
<211>  11
<212>  PRT
<213>  Artificial

<220>
<223>  Artificial integrin binding RGD comprising peptide

<400>  6

Glu Ala Cys Cys Arg Gly Asp Cys Val Cys Glu
1               5                   10


<210>  7
<211>  11
<212>  PRT
<213>  Artificial

<220>
<223>  Artificial integrin binding RGD comprising peptide

<400>  7

Ser Arg Leu Cys Arg Gly Asp Cys Val Asp Ala
1               5                   10


<210>  8
<211>  11
<212>  PRT
<213>  Artificial

<220>
<223>  Artificial integrin binding RGD comprising peptide

<400>  8

Ala Arg Val Cys Arg Gly Asp Cys Val Asp Gln
1               5                   10


<210>  9
<211>  11

<212>  PRT
<213>  Artificial

<220>
<223>  Artificial integrin binding RGD comprising peptide

<400>  9

Ala Arg Ser Cys Arg Gly Asp Cys Val Glu Ala
1               5                   10


<210>  10
<211>  11
<212>  PRT
<213>  Artificial

<220>
<223>  Artificial integrin binding RGD comprising peptide

<400>  10

Ala Arg Tyr Cys Arg Gly Asp Cys Phe Asp Gly
1               5                   10


<210>  11
<211>  11
<212>  PRT
<213>  Artificial

<220>
<223>  Artificial integrin binding RGD comprising peptide

<400>  11

Ala Leu Tyr Cys Arg Gly Asp Cys Val Asp Gln
1               5                   10


<210>  12
<211>  11
<212>  PRT
<213>  Artificial

<220>
<223>  Artificial integrin binding RGD comprising peptide

<400>  12

Ala Phe Leu Cys Arg Gly Asp Cys Val Asp Phe
1               5                   10


<210>  13
<211>  11
<212>  PRT
<213>  Artificial

<220>
<223>  Artificial integrin binding RGD comprising peptide

<400>  13

Leu Ser Gly Cys Arg Gly Asp Cys Phe Gln Gln
1               5                   10

<210>  14
<211>  11
<212>  PRT
<213>  Artificial

<220>
<223>  Artificial integrin binding RGD comprising peptide

<400>  14

Leu Ser Ala Cys Arg Gly Asp Cys Gln Arg Val
1               5                   10

<210>  15
<211>  11
<212>  PRT
<213>  Artificial

<220>
<223>  Artificial integrin binding RGD comprising peptide

<400>  15

Phe Pro Gly Cys Arg Gly Asp Cys Ser Gln Glu
1               5                   10

## Description

The present invention relates to novel RGD comprising polypeptides, compositions and complexes comprising them as well as their use, in particular for the therapy of proliferative diseases, immune diseases, infectious diseases, vascular diseases, rheumatoid diseases, diseases, in which cells in or adjacent to a disease site express α_{V}β₃ and/or α_{V}β₅ integrin and inflammatory diseases.

The delivery of drugs to only specific sites within the body is one central goal of targeted drug therapy. The concept depends on the differential expression of certain target structures only in a subpopulation of tissues or cell types of the body. Therefore, many groups have tried to elucidate differences in the expression of cell surface markers among different tissue types or between the healthy and diseased state of a tissue or cell. Targeted drug delivery is an attractive concept in particular for cancer chemotherapy, which usually administers highly toxic substances systemically. It is believed that targeted drug delivery to primarily to the tumor cells or at least primarily into the vicinity of the tumor cells would allow to either increase the amount of chemotherapeutic, which can be administered at the same level of systemic toxicity but with an increased effect at the tumor site, or to decrease the amount of chemotherapeutic, which is administered thus lowering the systemic toxicity while still eliciting the same effect at the tumor site.

Of many differentially expressed target structures identified so far, α_{V}β₃ and α_{V}β₅ integrins represent particular attractive target structures since they are upregulated in proliferating endothelial cells of the tumor vasculature. Targeting of the tumor vasculature, thus, represents a promising new approach for targeted cancer therapy (Matter (2001) Drug Discov. Today 6:1005-1024). Vascular targeting agents are designed to deliver cytotoxic, anti-angiogenic, procoagulant, or proapoptotic substances specifically to the vasculature of tumors. The employed ligands recognize structures associated with tumor blood vessels, i.e. proteins expressed by endothelial cells or associated with the extracellular matrix (Thorpe *et al*., (2003) Cancer Res. 63:1144-1147; Halin *et al*. (2001) News Physiol. Sci. 16:191-194). Targeting of the vasculature has several advantages over targeting of tumor cells (Augustin (1998) Trends.

Pharmacol. Sci. 19:216-222). There are no physiological barriers as endothelial cells are easily accessible to circulating carrier systems and penetration into the tumor is not necessary. Destruction of few capillary endothelial cells affects a large number of tumor cells depending on them. Since all solid tumors are dependend on neovascularization to grow beyond a few millimeters in diameter, this approach should be broadly applicable. Finally, endothelial cells are genetically stable and do not become resistant to the therapy due to mutation.

In addition α_{V}β₃ and α_{V}β₅ integrins are found on different tumor cells including metastatic melanoma cells (Conforti *et al*. (1992) Blood 80:437-446; Gehlsen *et al*. (1992) Clin. Exp. Metastasis 10:111-120; Seftor *et al*. (1999) Cancer Metastasis Rev. 18:359-375 and Varner & Cheresh (1996) Curr. Opin. Cell. Biol. 8: 724-730; Nahde et al, (2001) J. Gene Med. 3: 353-361). Targeting to α_{V}β₃ and α_{V}β₅ integrins can be achieved by RGD-containing peptides, with a variety of linear and cyclic RGD-containing peptides (DeNardo *et al*. (2000) Cancer Biother. Radiopharm. 15:71-79; Pasqualini *et al*. (1997) Nat. Biotechn. 15:542-546). Several of these RGD-peptides have already been used to deliver radionuclide, proteins, cytotoxic drugs or viral and non viral carrier systems to integrin-expressing cells *in vitro* and *in vivo* (Arab *et al*. (1998) Science 279:377- 380; Schraa *et al*. (2002) Int. J. Cancer 102:469-475; Jansen *et al*. (2002) Cancer Res. 62:6146-6151; Erbacher *et al*. (1999) Gene Ther. 6:138-145; Müller *et al*. (2001) Cancer Gene Ther. 8:107-117 and Wicknam *et al*. (1997) J. Virol. 170:8221- 8229; Nahde et al, (2001) J. Gene Med. 3: 353-361).

One problem when designing RGD-comprising integrin binding peptides lies in a lack of conformational stability of the resulting peptides. This problem has been addressed, for example, in US 5,880,092 by cyclization of RGD-containing peptides sequences (see also, for example, Koivunen, *et al*. (1995) Biotechnology 13:265 - 280, U.S. 5,880,092; US 5,981,468; US 6,020,460; US 5,906,975; US 5,985,827; US 5,827,821; and US 6,353,090). Other approaches for providing conformational stabile RGD-comprising integrin binding peptides involved the introduction of four cysteine residues which are oxidized in a step wise fashion to form two intermolecular bridges preferable in the 1 - 4 and 2 - 3 conformation as described by, for example, Assa-Munt *et al*. (2001) Biochemistry 40:2373-2378. However, the generation of such stabilized peptides involves two separate oxidation steps, which is cumbersome.

Thus, there is still a need in the art to identify novel RGD-comprising peptides, which exhibit high integrin binding affinity and/or which are easy to produce in a stabilized form. To this end the present inventors have identified novel RGD-comprising peptides.

Therefore, in one aspect of the present invention a polypeptide is provided comprising at least one binding peptide having an amino acid sequence as indicated by the general formula (I) X₁X₂X₃Y₁Y₂Y₃Y₄Y₁X₄X₅X₆ or by the general formula (II) X₇X₈X₉Y₁Y₂Y₃Y₄Y₁X₁₀Y₁X₁₁, in which:
Y₁ is Cys
Y₂ is Arg
Y₃ is Gly
Y₄ is Asp
X₁ is Ala, Leu, Phe or Ser, in particular Ala or Leu;
X₂ is Arg, Leu, Phe, Pro, or Ser, in particular Arg or Ser;
X₃ is Ala, Gly, Leu, Ser, Tyr, or Val, in particular Gly, Leu, or Tyr;
X₄ is Gln, Phe, Ser, or Val, in particular, Phe or Val;
X₅ is Arg, Asp, Glu, or Gln, in particular Asp or Gln;
X₆ is Ala, Gln, Glu, Gly, Phe, or Val, in particular Ala, Gln or Gly;
X₇ is Glu, Phe, Pro, or Val, in particular Glu or Val;
X₈ is Ala or Cys, in particular Cys;
X₉ is Asp, Cys, Gln, or His, in particular Gln;
X₁₀ is Leu, Phe, or Val, in particular Leu; and
X₁₁ Gln, Phe, Pro, or Val, in particular Pro,
wherein X₁, X₂, X₃, X₄, X₅, X₆, X₇, X₈, X₉, X₁₀, X₁₁, Y₁, Y₂, Y₃, Y4 are independently of each other the D or L amino acid or the amino acid residue mimetic of the respectively indicated amino acid;
or said amino acid sequence, which lacks 1 or 2, preferably 1, amino acid(s) from the N-terminus or C-terminus or 1 amino acid from the N- and C-terminus.

Polypeptides according to formula I have a particular high binding affinity for α_{V}β₃ and/or α_{V}β₅ integrins, while polypeptides according to structure (II) have a high binding activity albeit they require only one oxidation step for stabilizing the RGD motive.

The term "amino acid residue mimetic" refers to a moiety, that conformationally and/or functionally serves as a substitute for a particular amino acid in a peptide compound without adversely interfering to a significant extent with the function of the peptide (e.g., interaction of the peptide with α_{V}β₃ and/or α_{V}β₅ integrins) and shall include pseudomimetics and secondary structure mimetics (as described in Peptides: Chemistry and Biology by Norbert Sewald (Author), Hans-Dieter Jakubke (Author), Publisher: Wiley-VCH). By substituting amino acids with their mimetics the peptide or polypeptide retains many of the biologically important structural features of its parent while possessing a reduced or modified peptidic character.

Amino acid residue mimetics do not necessarily have to make the same contacts as the amino acid they replace it is often sufficient if they do not alter the overall conformation in such that the function, e.g. the binding to α_{V}β₃ and/or α_{V}β₅ integrins, is not significantly affected. In some circumstances, substitution with an amino acid residue mimetic may actually enhance properties of the peptide (e.g., interaction of the peptide with α_{V}β₃ and/or α_{V}β₅ integrins or stability or oral uptake of the peptide). Amino acid residue mimetics for the amino acids making up the binding peptide of the polypeptide of the present invention are well known in the art and approaches to designing peptides including amino acid residue mimetic are also well known in the art. For example, see Farmer, P. S. in Drug Design (E. J. Ariens, ed.) Academic Press, New York, 1980, vol. 10, pp. 119-143; Ball. J. B. and Alewood, P. F. (1990) J. Mol. Recognition 3:55; Morgan, B. A. and Gainor, J. A. (1989) Ann. Rep. Med. Chem. 24:243-252; and Freidinger, R. M. (1989) Trends Pharmacol. Sci. 10:270; A. D. Abell (editor) Advances in peptidomimetics and amino acid mimics, JAI Press, USA, Volume 1, 1997.

The strength of the interaction of a polypeptide of the present invention comprising one or more amino acid residue mimetics can be determined by a wide variety of assays known in the art and which are suitable to determine the binding between two chemical entities and comprise without limitation the binding assays described in the examples as well as methods as ELISA and plasmon resonance. Polypeptides comprising one or more amino acid residue mimetics are still considered to show no significant decrease in binding if they posses at least 30%, preferably at least 50%, more preferably at least 75% and most preferably at least 100% of the binding strength of a polypeptide comprising a binding peptide entirely made up of L amino acids.

Particularly preferred amino acid residue mimetics for the amino acids making up the binding peptide comprise but are not limited to:
Ala: alanine substituted at the carbon or nitrogen residue with alkl, cycloalkyl or aryl;
Asp: aryl and alkyl esters of aspartic acid;
Cys: cystein substituted at the sulfur atom with alkyl, alkenyl or phenyl optionally substituted with halo, cyano, nitro, haloalkyl, amino, aminoalkyl, dialkylamino, alkyl, alkoxy, haloalkoxy, carboxyl, carboalkoxy, alkylcarboxamide, arylcarboxamide, alkylthio or haloalkylthio;
Gln: glutamine substituted at the nitrogen atom with alkyl, alkenyl, alkynyl, alkoxyalkyl, alkylthioalkyl, alkylaminoalkyl, dialkylaminoalkyl, carboxyalkyl, alkoxycarbonyl alkyl or cycloalkyl, bicycloalkyl, cycloalkyl alkyl, bicycloalkyl alkyl or fused aryl- cycloalkyl alkyl optionally comprising 1 or more heteroatoms selected from N, O and S;
Glu: aryl and alkyl esters of glutamic acid;
Gly: glycine substituted at the carbon or nitrogen residue with alkl, cycloalkyl or aryl, in particular t-butyl glycine;
Leu: isoleucine;
Phe: homo-phenylalanine, dehydro-phenylalanine, indoline-2- carboxylic acid; tetrahydroisoquinoline-2-carboxylic acid optionally substituted with halo, cyano, nitro, haloalkyl, amino, aminoalkyl, dialkylarino, alkyl, alkoxy, haloalkoxy, carboxyl, carboalkoxy, alkylcarboxamide, arylcarboxamide, alkylthio or haloalkylthio;
Ser: serine substituted with alkyl or aryl;
Tyr: tyrosine substituted with alkyl or aryl;
Val: norvaline or norleucine.

The term "polypeptide" as used in the present invention refers to a chain of naturally and/or non-naturally occurring amino acids , including for example D amino acids, linked via peptide bonds to each other. The length of the amino acid chain is not particular limited as long as it comprises at least the binding peptide having an amino acids sequence as specified above. Preferably the polypeptide still shows no significant decrease of the interaction with its target, e.g. α_{V}β₃ and/or α_{V}β₅ integrins as defined above.

In a preferred embodiment the polypeptide of the present invention comprises a least one binding peptide having an amino acids sequence as indicated by the general formula (I) X₁X₂X₃Y₁Y₂Y₃Y₄Y₁X₄X₅X₆ or by the general formula (II) X₇X₈X₉Y₁Y₂Y₃Y₄Y₁X₁₀Y₁X₁₁, in which
Y₁ is Cys
Y₂ is Arg
Y₃ is Gly
Y₄ is Asp
X₁ is Ala or Leu;
X₂ is Arg or Ser;
X₃ is Gly, Leu or Tyr;
X₄ is Phe or Val;
X₅ is Asp or Gln;
X₆ is Ala, Gln, or Gly;
X₇ is Glu or Val;
X₈ is Cys;
X₉ is Gln;
X₁₀ is Leu; and
X₁₁ is Pro,
wherein X₁, X₂, X₃, X₄, X₅, X₆, X₇, X₈, X₉, X₁₀, X₁₁, Y₁, Y₂, Y₃, Y₄ are independently of each other the D or L amino acid or the amino acid residue mimetic of the respectively indicated amino acid or said amino acid sequence, which lack 1 or 2, preferably 1, amino acid(s) from the N-terminus or C-terminus or 1 amino acid from the N- and C-terminus.

In an even more preferred embodiment the polypeptide of the present invention comprises at least one binding peptide with an amino acid sequence as indicated in SEQ ID NOs. 1 to 15.

In a further preferred embodiment the polypeptide of the present invention has a length of preferably 500, 450, 400, 350, 300, 250, 200, 100, 90, 80, 70, 60, 50, 40, 30, 20, 19, 18, 17, 16, 15, 14, 13, 12, 11, 10 or 9 amino acids. Particular preferred are polypeptides with a length of between about 10 to 20 amino acids.

In the further preferred embodiment the polypeptide of the present invention can comprise additional encoded proteins, which can be essentially any desired protein or protein fragment, however, in preferred embodiments of the invention the polypeptide comprising the binding peptide further comprises a protein selected from the group consisting of a cytokine, a chemokine, a growth factor, an adhesion molecule, an antibody light and/or heavy chain, a single chain antibody, a toxin, an enzyme, a receptor ligand, a lytic peptide, a membrane insertion sequence and a fluorescent protein or fragment thereof.

Such fusion polypeptides can provide an efficient way of recruiting the one or more additionally encoded protein or fragment thereof to cells expressing α_{V}β₃ and/or α_{V}β₅ integrins. In particular in cases in which a cytokine, a chemokine or a prodrug thereof or a toxin is delivered to a cell expressing one of the mentioned integrins a localized cytotoxic effect can be obtained.

In another preferred embodiment of the present invention the polypeptide is attached to at least one chemical moiety. The term "attached" as used through out this specification means a direct or indirect, covalent or non-covalent bond and connection, respectively, between a polypeptide of the present invention and another chemical entity. The term "chemical moiety" or "entity" as used interchangeably herein is not limited to a particular type of a chemical substances, however, in a preferred embodiment the chemical moiety is selected from the group consisting of a spacer, a marker, a tag, and a lipid and in particular a phospholipid, a drug, a capping group and a spacer attached to a second chemical moiety.

A "capping group" within the meaning of the present invention is a chemical moiety which protects the molecule to which it is attached from, for example, chemical or enzymatic degradation. The capping group or groups can be attached directly to the polypeptide of the present invention or to any other chemical moiety, which is itself attached to the polypeptide of the present invention. In certain preferred aspects of the present invention in which the N- and/ or C-terminus of the polypeptide of the present invention is not attached to another chemical moiety like, for example, a spacer and would otherwise be in its "free" form, i.e. ―COOH and/or -NH₂, a capping group is attached to one or both ends to avoid or minimize degradation by, for example, exoproteinases or the like.

The capping groups of the present invention have in a preferred embodiment one of the following structures:

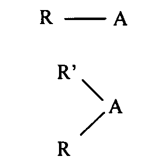

Wherein A is an amino acid or an amino acid residue mimetic of the polypeptide of the present invention or any other chemical moiety attached to the polypeptide of the present invention and R and R' each independently have the meaning: -C(O)R₁, - C(O)NHR₁, -S(O)2R1, -C(O)OR₁, CR₁ or R₁,
wherein R₁ can have the meaning H; linear or branched alkyl, in particular lower alkyl (C₁, C₂, C₃, C₄, and C₅, e.g. methyl, ethyl, n-propyl, isopropyl, n-butyl, isobutyl, tert-butyl, n-pentyl or iso-pentyl); substituted linear or branched alkyl, in particular lower substituted alkyl; linear or branched alkenyl, in particular lower alkenyl (C₂, C₃, C₄ and C_{5,}, e.g. ethenyl, 1-propenyl, 2-propenyl, iso-propenyl, 1-butenyl, 2-butenyl, 3-butenyl; substituted linear or branched alkenyl, in particular lower substituted alkenyl; linear or branched alkynyl, in particular lower alkynyl (C₂, C₃, C₄ and C₅); substituted linear or branched alkynyl, in particular lower substituted alkynyl; linear or branched alkanol, in particular lower alkanol (C₁, C₂, C₃, C₄, and C₅); linear or branched alkanal, in particular lower alkanal (C₁, C₂, C₃, C₄, and C₅, e.g. COH, CH₂COH, CH₂CH₂COH; aryl, in particular phenyl; substituted aryl, in particular substituted aryl; haloalkyl, in particular lower haloalkyl (C₁, C₂, C₃, C₄, and C₅); haloalkoxy, in particular lower haloalkoxy (C₁, C₂, C₃, C₄, and C₅); heteroaryl optionally comprising 1 to 4 heteroatoms selected from N, O, S; substituted heteroaryl optionally comprising 1 to 4 heteroatoms selected from N, O, S; aryl, in particular C₅ to C₁₂); alkylaryl, in particular C₅ to C₁₂, e.g. benzyl, isoquinolinyl, quinolinyl, naphthyl; substituted alkylaryl, in particular C₅ to C₁₂, e.g. substituted benzyl; alkylheteroaryl optionally comprising 1 to 4 heteroatoms selected from N, O, S optionally comprising 1 to 4 heteroatoms selected from N, O, S; substituted alkylheteroaryl optionally comprising 1 to 4 heteroatoms selected from N, O, S; aminoalkyl, in particular C₁, C₂, C₃, C₄ and C₅, e.g. -NHCH₃, -NHCH₂CH3, - N(CH₃)₂; substituted aminoalkyl; dialkylamino; aminoketone, in particular -NHCOCH₃; substituted aminoketone; aminoaryl, in particular ―NH-Ph; substituted aminoaryl, in particular substituted ―NH-Ph; carboxyl, carboalkoxy; alkylcarboxamide; cycloalkyl; alkylcycloalkyl; CN; NH₂; Halogen, in particular F, Cl, and Br; if the residues mentioned above are substituted they are preferably mono, di, or tri substituted with a substituent selected from the group of halogen, in particular F, Cl, and Br, NH₂, NO₂, OH, SH, NH, CN, aryl, alkylaryl, heteroaryl, akylheteroaryl, COH or COOH;
R and R' form a ring comprising 5-7 atoms selected from C, N, S and O; or
R and R' are independently toluenesulfonyl, methanesulfonyl, FMOC or (+)-menthyloxy-CO-.

The term "spacer" refers to a chemical moiety, which serves the purpose of providing the accessibility of the binding peptide even when attached to other chemical moieties which might otherwise sterically hinder the binding of the polypeptides of the present invention to its target structures. Spacer within the meaning of the present invention are a linear extension of at least 0.5 nm preferably the spacer has a linear extension of between 1 and 10 nm and even more preferably between 2 and 5 nm. The spacer is preferably a linear or branched saturated or unsaturated carbohydrate chain. The carbohydrate chain preferably comprises multimeric repeats of a monomeric building block. Depending on the length of the respective monomeric building block between 2 and 10 multimeric repeats of the monomeric building blocks are preferred. In preferred embodiments the spacer is hydrophilic. The spacer can comprise a functional group which allows attachment to the polypeptides of the present invention on one terminus and another functional group on the other terminus, which allows attachment of the spacer to another chemical moiety.

Preferred spacers are bifunctional molecules in particular bifunctional polyethylene or polypropylene glycol derivatives comprising preferably between about 1 and 40 repeat units, oligopeptides comprising natural and/or synthetic amino acids and preferably between 1 to 40 preferably 2 to 20 and more preferably 2 to 10 amino acids. A particular preferred building block of a spacer of the present invention is 8-amino-3, 6-dioxatanoic acid (doo) and spacers comprising between 1 to 10 repeat units of doo are preferred. Spacers comprising between 2 and 5 doo units being more preferred and spacers comprising 3 doo units being most preferred. In the context of liposomes it has been discovered that there is an optimal length of the spacer, which is between 2 and 5 nm. While spacers with a length of less than 0.5 nm will in most cases not provide enough distance from, for example, the liposomal surface to which the polypeptides of the present invention has been attached to allow efficient interaction, i. e. binding, between the binding peptides of the present invention and integrin bearing cells. On the other hand spacers, which are longer than 10 nm show an increasing "floppiness" which is also detrimental to the binding to cells.

The term "marker" refers to a chemical moiety which is directly or indirectly detectable by analytical methods including measurement of fluorescence, nuclear magnetic resonance, computer tomography or scintigrams and comprises without limitation electron dense molecules, paramagnetic molecules, superparamagnetic molecules, radioactive molecules like, for example, ¹³N, ¹⁵O, ¹⁸F, ⁵¹Gr, ⁵⁴Fe, ⁶⁰Co, ⁶⁷Ga, ⁷⁵Se, ^{99m}Tc, ¹¹¹In, ^{112m}Ag, ^{113m}In, ¹²³I, ¹³³Xe, ¹⁴⁸Au, ³⁵S, ³³P, ³²P, or ¹¹C, non-radioactive isotopes, which include, for example, ²H and ¹³C, and fluorescent molecules or molecules generating fluorescence or light emission like, for example, green fluorescent protein, luciferase, and a variety of fluorescent dies all of which are well known to someone of skill in the art.

The term "tag" refers to chemical moieties, which allow purification of the polypeptides or complexes comprising the polypeptides of the present invention like, for example, biotin, Chitin-tag, Myc-tag, His-tag or the like, which are all well known in the art.

In a preferred embodiment the polypeptide of the present invention is attached directly or indirectly to a lipid. The type of lipid to which the polypeptide can be attached is not particular limited. However, in preferred embodiments the respective lipid can be inserted or incorporated into lipid-based carriers like, for example, liposomes or virosomes. Particularly suitable lipids are glycerides, glycerophospholipides, glycerophosphinolipids, glycerophosphonolipids, sulfolipids, sphingolipids, phospholipids, isoprenolides, steroids, stearines, steroles, and/or carbohydrate containing lipids. Particularly preferred lipids for the attachment of the polypeptides of the present invention are phospholipids preferably phosphatidylcholine (PC), phosphatidylserine (PS), and phosphatidylethanolamine (PE), in particular distearoylphosphatidyl (DSPE) or alpha-(dipalmitoylphosphatidyl (DPP)) which are often included in liposomes used for delivery of drugs.

In another embodiment the lipid attached to the polypeptide of the present invention is selected from the group consisting of N-caproylamine-PE, N-dodecanylamine-PE, phophatidylthioethanol, N-[4-(p-maleimidomethyl)cyclohexane-carboxamide-PE (N-MCC-PE), N-[4-(p-maleimidophenyl)butyramide]-PE (N-MPB), N-[3-(2-pyridyldithio)propionate]-PE (N-PDP), N-succinyl-PE, N-glutaryl-PE, N-dodecanyl-PE, N-biotinyl-PE, N-biotinyl-cap-PE, phosphatidyl-(ehtylene glycol), PE-polyethylene glycol (PEG)-carboxylic acid, PE-PEG-maleimide, PE-PEG-PDP, PE-PEG-amine, PE-PEG-biotin, PE-PEG-HNS, dipalmitoyl-glycerosuccinyl-lysine, alpha-methoxy-omega-(1,2-dioctadecenoyloxy glyceryl) (DO), alpa-methoxy-omega-(1,2-ditetradecenoyloxy glyceryl) (DT).

In a further preferred embodiment the polypeptide of the present invention is attached to at least one drug. The term "drug" encompasses any therapeutically active compound and in particular compounds are selected from the group consisting of immunosuppressants, immunostimulants, antibiotics, antiinfectives, antiallergics, cytostatics, cytotoxic agents and prodrugs thereof, mitogens, chemokines, cytokines, dermatics and/or physiological or pharmacological inhibitors of mitogens, chemokines or cytokines. In preferred embodiments the drug is attached to the polypeptide of the present invention in such a way that it is releasable and preferably the release of the drug is primarily effected in the tissues or areas of the body to which the polypeptide of the present invention binds i. e. primarily in tumor endothelium or tumors. Particularly preferred means of attachment of the drug are short polypeptide stretches which are cleavable, for example, by enzymes which are released at the target site. Thus, preferably the drugs are released in the tumor endothelium or in tumors. Enzymes of this type include, for example metalloproteinases. Such releasable connections are known in the art and can be selected to provide a further specificity on top of the specificity already achieved by the target specific binding of the polypeptides of the present invention. The inclusion of such a cleavage site is particularly desirable, in cases in which a drug attached to the polypeptides of the present invention exerts a cytotoxic and/or growth inhibitory effect on cells once it is released.

In a preferred embodiment of the present invention using above described spacer the spacer is attached at one side to the polypeptide of the present invention and on the other side to a second chemical moiety. Preferably the second chemical moiety is selected from the group consisting of a drug, a marker, a tag and a lipid. In this context the terms "drug", "marker", "tag", and "lipid", have the meaning and preferred meanings as outlined above.

A further aspect of the present invention is a polynucleotide encoding at least one polypeptide of the present invention comprising a binding peptide which has an amino acid sequence as outlined above. The nucleic acid molecules of the invention can be DNA, cDNA, genomic DNA, synthetic DNA or RNA and can be double-stranded or single-stranded, the sense and/or antisense strand. The polynucleotide molecules of the invention can contain any sequence which due to the degeneracy of the genetic code, encode a polypeptide comprising a binding peptide having an amino acid sequence as outlined above and preferably one of the amino acid sequences indicated in SEQ ID NOs. 1-15. In addition the nucleic acid molecules of the present invention are not limited to coding sequences, e.g. they can include none-coding sequence that can be located upstream or downstream from a sequence encoding the polypeptides of the present invention.

The polynucleotide molecules of the invention can be synthesized *in vitro*, for example, by phosphoramidite-based synthesis or can be obtained from a cell such as a bacterial cell, or mammalian cell.

A further aspect of the present invention is a vector containing the polynucleotide of the present invention. The term "vector" as used herein refers to a means for introducing the polynucleotides of the present invention into cells in particular for maintenance and propagation. In preferred embodiments the vector of the present invention comprises plasmids, phagemids, phages, cosmids, artificial mammalian or yeast chromosomes, knock-out or knock-in constructs, viruses in particular adeno-viruses, vaccinia-viruses, attenuated vaccinia-viruses, canary pox viruses, lentiviruses (Chang, L. J. Gay, E. E. (2001) Curr. Gene Therap. 1: 237-251), Herpes viruses in particular Herpes simplex virus (HSV-1) (Carlezon, W. A. *et al*. (2000) Crit. Rev. Neurobiol.), baculovirus, retrovirus, adeno-associated virus (AAV; Carter, P. J. and Samulski, R. J. (2000) J. Mol. Med. 6: 17-27), rhinovirus, human immune deficiency virus (HIV), Filovirus and engineered versions thereof (see, for example, Cobinger G. P. *et al*. (2001) Nat. Biotechnol. 19: 225-230), virosomes, "naked" DNA, liposomes and nucleic acid coated particles, in particular gold spheres. Particular preferred are viral vectors like adeno-viral vectors or retro-viral vectors (Lindemann *et al*. (1997) Mol. Med. 3: 466 - 476 and Springer *et al*. (1998) Mol. Cell. 2: 549 - 258). Liposomes are in general small unilamellar or multilamellar vesicles made of cationic, neutral and/or anionic lipids, for example, by ultrasound treatment of lipid suspensions. The DNA can, for example, be ionically bound to the surface of the liposome or internally enclosed in the liposome. Suitable lipid mixtures are known in the art and comprise for example DOTMA (1, 2-dioleyloxypropyl-trimethylamoniumbromid) and DPOE (dioleylphosphatidyl-ethanolamine) which both have been use for transfection or transduction of a variety of cell lines.

Nucleic acid coated particles are another means for the introduction of nucleic acids into cells using so called "gene-guns" which allow the mechanical introduction of particles into the cells. Preferably, the particles itself are inert and, therefore, are in a preferred embodiment made out of gold spheres.

In a further aspect the polynucleotide of the present invention is operatively linked to one or more expression control sequences allowing expression in prokaryotic and/or eukaryotic host cells. The "expression control sequences" are preferably transcriptional/translational regulatory elements, which include but are not limited to inducible and non-inducible, constitutive, cell cycle regulated, metabolically regulated promoters, enhances, operators, silencers, repressors and other elements that are known to those skilled in the art and that drive or otherwise regulate gene expression. Examples of regulatory elements directing constitutive expression include, promoters transcribed by RNA polymerase III like, e.g., promoters for the snRNA U6 or scRNA 7SK gene; viral promoter and activator sequences derived from, e.g., NBV, HCV, HSV, HPV, EBV, HTLV, CMV, SV40, MMTV or HIV, in particular the cytomegalovirus hCMV immediate early gene, the early or late promoters of SV40 adenovirus directing inducible expression like, for example, CUP-1 promoter, the tet-repressor as employed, for example, in the tet-on or tet-off systems, the lac system, the trp system; tissue specific expression elements; cell cycle specific expression elements like, for example, cdc2, cdc25C or cyclin A; or the TAC system, the TRC system, the major operator and promoter regions of phage A, the control regions of fd coat protein, the promoter for 3-phosphoglycerate kinase, the promoters of acid phosphatase, and the promoters of the yeast α- or a-mating factors.

As used herein, "operatively linked" means incorporated into a genetic construct so that expression control sequences effectively control expression and/or translation of a coding sequence of interest.

Another aspect of the present invention is a host cell genetically engineered with the polynucleotide or the vector as outlined above. The host cells that may be used for purposes of the invention include but are not limited to prokaryotic cells such as bacteria (for example, *E. coli* and *B. subtilis*), which can be transformed with, for example, recombinant bacteriophage DNA, plasmid DNA, or cosmid DNA expression vectors containing the polynucleotide molecules of the invention; simple eukaryotic cells like yeast (for example, *Saccharomyces* and *Pichia*), which can be transformed with, for example, recombinant yeast expression vectors containing the polynucleotide molecule of the invention; insect cell systems like, for example, Sf9 of Hi5 cells, which can be infected with, for example, recombinant virus expression vectors (for example, baculovirus) containing the polynucleotide molecules of the invention; Xenopus oocytes, which can be injected with, for example, plasmids; plant cell systems, which can be infected with, for example, recombinant virus expression vectors (for example, cauliflower mosaic virus (CaMV) or tobacco mosaic virus (TMV)) or transformed with recombinant plasmid expression vectors (for example, Ti plasmid) containing polynucleotide molecules of the invention; or mammalian cell systems (for example, COS, CHO, BHK, HEK293, VERO, HeLa, MDCK, Wi38, and NIH 3T3 cells), which can be transformed with recombinant expression constructs containing, for example, promoters derived, for example, from the genome of mammalian cells (for example, the metallothionein promoter), from mammalian viruses (for example, the adenovirus late promoter and the vaccinia virus 7.5K promoter) or from bacterial cells (for example, the tet-repressor binding its employed in the tet-on and tet-off systems). Also useful as host cells are primary or secondary cells obtained directly from a mammal and transfected with a plasmid vector or infected with a viral vector. Depending on the host cell and the respective vector used to introduce the polynucleotide of the invention the polynucleotide can integrate, for example, into the chromosome or the mitochondrial DNA or can be maintained extrachromosomally like, for example, episomally or can be only transiently comprised in the cells.

A further aspect of the present invention is a transgenic non-human animal containing a polynucleotide, a vector and/or a host cell as described above. The animal can be a mosaic animal, which means that only part of the cells making up the body comprise polynucleotides, vectors, and/or cells of the present invention or the animal can be a transgenic animal which means that all cells of the animal comprise the polynucleotides and/or vectors of the present invention or are derived from a cell of the present invention. Mosaic or transgenic animals can be either homo- or heterozygous with respect to the polynucleotides of the present invention contained in the cell. The animals can in principal be any animal, preferably, however, it is a mammal, selected from the group of non-human primate, horse, bovine, sheep, goat, pig, dog, cat, rabbit, mouse, rat, guinea pig, hamster, or gerbil.

The polypeptides of the invention comprising binding peptides can be used directly as a therapeutic or in combination with additional substances and, therefore, the present invention in a further aspect relates to a composition comprising at least one polypeptide of the present invention and at least one further component selected from the group consisting of liposomes, virosomes, microsphere, niosomes, dendrimeres, stabilizers, buffers, excipient, additives.

Liposomes are single or multilamellar lipid vesicles of varying size. The size is preferable between 10 and 1000 nm and more preferably between 50 and 500 nm and most preferably between 80 and 200 nm. Virosomes are liposomes with a lipid composition closely resembling the lipid composition of viruses and which in preferred embodiments have proteins integrated into the membrane (Kaneda (2000) Adv. Drug. Deliv. Rev. 43:197-205). Microspheres are spherical particle with large size (up to 2 mm) and rigid morphology containing a core substance (Ravi & Kumar (2000) J. Pharm. Sci. 3:234-258). Niosomes are non-ionic surfactant vesicles (Baillie et al. (1985) J. Pharm. Pharmacol. 37:863-868). Dendrimers are synthetic, highly branched, monodisperse macromolecules of nanometer dimensions (Patri et al. (2002) Curr. Opin. Chem. Biol. 6:466-471). Buffers comprised in the composition of the present invention can be any physiological buffer substances including, for example phosphate buffer or Hepes.

Excipients which facilitate production and administration of the compositions of the present invention are the art known excipients and include, for example, alginates, calcium carbonate, dextrose, fructose, lactose, maltose, maltodextrin, and the like. Stabilizers are also known in the art and comprise, for example, α-tocopherol and various carbohydrates.

In a preferred embodiment of the compositions of the present invention the polypeptide is integrated into or attached to a liposome, virosome, microsphere, niosome or dendrimer, which allows the respective entity to be target to sites and tissues in the body expressing α_{V}β₃ and/or α_{V}β₅ integrins. Polypeptides can be attached to one of the components, which are used for the generation of liposomes, virosomes, microsphere, niosomes, or dendrimers prior, during or after formation of the respective structure. In particular for liposomes and virosomes it is envisioned that polypeptides of the present invention which are linked to a lipid either with or without an intervening spacer as defined above are integrated into the lipid mono or multilayer of the liposome or virosome. In preferred embodiments the polypeptide is primarily comprised on the outer surface of the respective liposome or virosome to allow interaction of the polypeptides of the present invention with their targets preferably upon administration of the composition of the present invention to a patient. In preferred embodiments the polypeptide of the present invention will be attached to between about 0,1 mol% to about 10 mol% of all components which are used for the generation of the respective structure. This ranges are particular preferred for liposomes and virosomes.

In preferred embodiments the liposomes or virosomes of the present invention comprise lipids selected from the group consisting of glycerides, glycerophospholipides, glycerophosphinolipids, glycerophosphonolipids, sulfolipids, sphingolipids, phospholipids, isoprenolides, steroid, stearines, steroles, and carbohydrate containing lipids. In preferred embodiments the liposome or virosome comprises cholesterol (CH) and sphingomyelin (SM). More preferably cholesterol is comprised in relation to the total molar lipid composition of the liposome or virosome at a molar ratio of about 40 to about 60 mol% and more preferably of about 45 to about 55 mol% and most preferably of about 48 to about 52 mol%. SM on the other hand is preferably present in relation to the total molar lipid composition of the liposome at a molar ratio of about 10 to about 20 mol% and more preferably of about 11 to 18 mol% and most preferably of about 12 to about 16 mol%. In a particular preferred embodiment the liposome or virosome comprises in relation to the total molar lipid composition CH and SM at a molar ration of about 40 to about 60 mol% and of about 10 to about 20 mol%, respectively, and even more preferred at a molar ratio of about 45 to about 55 mol% and of about 11 to about 18 mol%, respectively and most preferably of about 48 to about 52 mol% and of about 12 to about 16 mol%, respectively.

The liposomes or virosomes which are comprised in the composition to the present invention contain (a) further lipid(s). This(These) is(are) preferably selected from the above indicated preferred lipids. In preferred embodiments at least one of the additional lipids is selected from PE and PC. In a particular preferred embodiment PE is present in the liposome or virosome of the present invention and in particular, if CH and SM are also present in the liposome or virosome. Preferably CH and SM are present together with PE in the above indicated preferred and particularly preferred ranges. PE itself is present in preferred embodiments in relation to the total molar lipid composition of about 5 to about 25 mol%, preferably about 10 to about 20 mol% and most preferably about 12 to about 18 mol%. Again in particular preferred embodiments CH, SM, and PE are present in above indicated preferred or particular preferred ranges.

In a further embodiment the additional lipids comprise PE and PC and in a preferred embodiment PE and PC are present in relation to the total molar lipid composition of the liposome or virosome at a molar ratio of about 5 to about 25 mol% and about 15 to about 40 mol%, respectively. The above ranges for PE are PC particular preferred, if the molar ratio of CH and SM are as indicated above.

In a further embodiment any of the components making up the membrane of the liposomes of the present invention can be attached to a further chemical moiety. The term chemical moiety is not particular limited. However, in preferred embodiments the chemical moiety is a targeting moiety as discussed in more detail below or a stabilizing moiety. Stabilizing moieties within the meaning of this invention increase the circulation time of the liposome once it is administered. Particular preferred stabilizing moieties are ganglioside GM1, phosphatidylinositol or PEG, particular preferred PEGs have a molecular mass between about 1,000 and about 10,000 g/mol, more preferably about 5,000 g/mol.

In a preferred embodiment the chemical moieties in particular the stabilizing moieties are attached to only a fraction of the molecules making up the membrane of the liposomes. It is preferred that between about 1 to about 20 mol% of the components of the liposomal membrane carry an attached chemical moiety, more preferably between about 3 and about 10 mol% and even more preferably about 5 mol%.

A preferred liposomal component for attachment of the chemical moiety, in particular for the stabilizing moiety is a lipid component. While different chemical moieties can be attached to different lipid components it is preferred that the chemical moiety(ies) is(are) attached to one or more of the phospholipids comprised within the liposome of the present invention. In a further preferred embodiment the one or more chemical moiety is attached to PE. In particular, if a stabilizing agent like, for example, PEG is used PE is used for attachment.

In addition to the attachment of stabilizing moieties detergents, proteins and peptides can be incorporated into the liposome for stabilizing the lipid bilayers of the liposomes of the present invention. Detergents which can be used as bilayer stabilizing components include, but are not limited to, Triton X-100, deoxycholate, octylglucoside and lyso-phosphatidylcholine. Proteins which can be used as bilayer stabilizing components include, but are not limited to, glycophorin and cytochrome oxidase. In preferred embodiments a liposome can comprise between 0.05 and 15 mol% of a stabilizing agent.

To exert a therapeutic effect the composition of the present invention can further comprise a drug. Preferably this drug is connected or attached directly or indirectly with a polypeptide of the present invention or comprised or attached to the liposomes, virosomes, microspheres, etc.

If a drug or diagnostic is comprised within a liposome it is particularly preferred that the drug or diagnostic is comprised in the interior of the liposome or in cases of lipophilic drugs also within or between the lipid bilayers. In preferred embodiments the drug is comprised within the liposome, virosome, microsome or niosome. A variety of methods are available in the prior art to "load" a liposome, virosome, microsome or niosome with a given drug or diagnostic. In its simplest form the drug and/or diagnostic is/are admixed with the lipid components during formation of the liposomes. Other passive loading methods include dehydration-rehydration (Kirby & Gregoriadis (1984) Biotechnology 2:979), revers-phase evaporation (Szoka & Papahadjopoulos (1978) Proc. Natl.Acad. Sci. USA 75:4194-), or detergent-depeletion (Milsmann et al. (1978) Biochim. Biophys. Acta 512:147-155). However, these techniques often lead to a substantial loss of drug during loading, which is a particular disadvantage in cases where the drug is expensive.

Other methodologies for encapsulating drugs and/or diagnostics include so called "remote loading" or "active loading" in which due to a gradient, for example, a pH or salt gradient between the exterior and the interior of a preformed liposome the drug and/or diagnostic is transported into the liposome along the gradient (see, for example Cheung et al. (1998) Biochim. Biophys. Acta 1414:205-216; Cullis et al. (1991) Trends Biotechnol. 9:268-272; Mayer et al. (1986) Chem. Phys. Lipids 40:333-345).

The passive and active loading techniques referred to above and other methods well known in the art can all without limitation employed by the skilled artisan. The most efficient method of loading for any given drug or diagnostic can be determined by routine experimentations by well established procedures. Variables which are typically adjusted are pH, temperature, salt type and concentration, type of buffer etc.

In a preferred embodiment the drugs and/or diagnostics are loaded by remote loading into the liposomes, virosomes, microsomes or niosomes, since this method offers a very low loss of the substance to be loaded. In a preferred embodiment a pH gradient is used for loading. Depending on the substance to be loaded the interior of the liposome will typically be acidified with respect to its exterior. Preferably the interior will have a pH between 1 and 6 prior to loading with the drug or diagnostic.

Particularly preferred drugs are selected from the group consisting of analgesics, antirheumatics, anthelminthics, antiallergics, antianemics, antiarrhythmics, antibiotics, angiogenesis inhibitors, antiinfectives, antidemenics (nootropics), antidiabetics, antidotes, antiemetics, antivertiginosics, antiepileptics, antihemorrhagics, antihypertonics, antihypotonics, anticoagulants, antimycotics, antitussiv agents, antiviral agents, beta-receptor and calcium channel antagonists, broncholytic and antiastmatic agent, chemokines, cytokines, mitogens, cytostatics, cytotoxic agents and prodrugs thereof, dermatics, hypnotics and sedatives, immunosuppressants, immunostimulants, peptide or protein drugs, in particular hormones and physiological or pharmacological inhibitors of mitogens, chemokines, or cytokines or their respective prodrugs. Of course it is also envisioned that a liposome of the invention comprises more than one drug at once. Numerous drugs for each category are known to the skilled artisan and are all included without limitation.

Since α_{V}β₃ and α_{V}β₅ integrins are primarily found on growing endothelial and in particular on tumor endothelial cells the polypeptide of the present invention allow the specific targeting of drugs, which can interfere with tumor growth and/or progression of the disease in a targeted manner, therefore, the composition of the present invention are particular suitable for treatment of hyperproliferative diseases associated with neovascularization. Consequently, particular preferred drugs are cytostatics and cytotoxic drugs. A large variety of such drugs are known in the art. Particular preferred cytostatics and cytotoxic drugs are selected from the group consisting of alkylating substances, anti-metabolites, antibiotics, epothilones, nuclear receptor agonists and antagonists, anti-androgenes, anti-estrogens, platinum compounds, hormones and antihormones, interferons and inhibitors of cell cycle-dependent protein kinases (CDKs), inhibitors of cyclooxygenases and/or lipoxygenases, biogeneic fatty acids and fatty acid derivatives, including prostanoids and leukotrienes, inhibitors of protein kinases, inhibitors of protein phosphatases, inhibitors of lipid kinases, platinum coordination complexes, ethyleneimenes, methylmelamines, trazines, vinca alkaloids, pyrimidine analogs, purine analogs, alkylsulfonates, folic acid analogs, anthracendiones, substituted urea, methylhydrazin derivatives, in particular acediasulfone, aclarubicine, ambazone, aminoglutethimide, L-asparaginase, azathioprine, bleomycin, busulfan, calcium folinate, carboplatin, carpecitabine, carmustine, celecoxib, chlorambucil, cis-platin, cladribine, cyclophosphamide, cytarabine, dacarbazine, dactinomycin dapsone, daunorubicin, dibrompropamidine, diethylstilbestrole, docetaxel, doxorubicin, enediynes, epirubicin, epothilone B, epothilone D, estramucin phosphate, estrogen, ethinylestradiole, etoposide, flavopiridol, floxuridine, fludarabine, fluorouracil, fluoxymesterone, flutamide fosfestrol, furazolidone, gemcitabine, gonadotropin releasing hormone analog, hexamethylmelamine, hydroxycarbamide, hydroxymethylnitrofurantoin, hydroxyprogesteronecaproat, hydroxyurea, idarubicin, idoxuridine, ifosfamide, interferon α, irinotecan, leuprolide, lomustine, lurtotecan, mafenide sulfate olamide, mechlorethamine, medroxyprogesterone acetate, megastrolacetate, melphalan, mepacrine, mercaptopurine, methotrexate, metronidazole, mitomycin C, mitopodozide, mitotane, mitoxantrone, mithramycin, nalidixic acid, nifuratel, nifuroxazide, nifuralazine, nifurtimox, nimustine, ninorazole, nitrofurantoin, nitrogen mustards, oleomucin, oxolinic acid, pentamidine, pentostatin, phenazopyridine, phthalylsulfathiazole, pipobroman, prednimustine, prednisone, preussin, procarbazine, pyrimethamine, raltitrexed, rapamycin, rofecoxib, rosiglitazone, salazosulfapyridine, scriflavinium chloride, semustine streptozocine, sulfacarbamide, sulfacetamide, sulfachlopyridazine, sulfadiazine, sulfadicramide, sulfadimethoxine, sulfaethidole, sulfafurazole, sulfaguanidine, sulfaguanole, sulfamethizole, sulfamethoxazole, co-trimoxazole, sulfamethoxydiazine, sulfamethoxypyridazine, sulfamoxole, sulfanilamide, sulfaperin, sulfaphenazole, sulfathiazole, sulfisomidine, staurosporin, tamoxifen, taxol, teniposide, tertiposide, testolactone, testosteronpropionate, thioguanine, thiotepa, tinidazole, topotecan, triaziquone, treosulfan, trimethoprim, trofosfamide, UCN-01, vinblastine, vincristine, vindesine, vinblastine, vinorelbine, and zorubicin, or their respective derivatives or analogs thereof. Several of the above indicated drugs are now administered simultaneously for cancer therapy and, consequently, it is also envisioned that more than one cytostatic and/or cytotoxic drug is comprised in a liposome of the present invention.

In another aspect the liposomes of the present invention can comprise anti-angiogenic drugs like, for example, fumagillin analogs, thalidomide, 2-methoxyestradiol, protein tyrosine kinase inhibitors. Such drugs are preferred for the treatment of diseases involving activated and/or proliferating endothelial cells For the treatment of diseases involving activated and/or proliferating endothelial cells, however, it is also possible to use cytostatic or cytotoxic drugs in particular those, which are indicated as preferred cytostatic or cytotoxic drugs above.

The term "immunosuppressant" comprises both substances which lower the activity of immune response as well as substances with an anti-inflammatory action, preferred examples are glucocorticoids, in particular beclomethasone, betamethasone, clocortolone, cloprednol, cortisone, dexamethasone, fludrocortisone, fludroxycortide, flumetasone, fluocinolone acetonide, fluocinonide, fluocortolone, fluorometholone, fluprednidene acetate, hydrocortisone, paramethasone, prednisolone, prednisone, prednylidene, pregnenolone, triamcinolone or triamcinolone acetonide, a cyclosporin, in particular cyclosporin A, mycophenolate mofetil, tacrolimus, rapamycin, FK 506, cycloheximide-N-(ethyl ethanoate), azathioprine, ganciclovir, an anti-lymphocyte globulin, ascomycin, myriocin, a pharmacological inhibitor of MAP kinases (especially a p38 inhibitor such as VX-745), caspase inhibitors, matrix metalloproteinase inhibitors, and/or methotrexate.

The term "immunostimulant" encompasses all substances, which influence the function of cells which are involved directly or indirectly in mediation of the immune response, and where the influence leads to an immune response. These cells include, for example, macrophages, Langerhans cells and other dendritic cells, lymphocytes, indeterminate cells, but also cells which do not themselves belong to the immune system but are involved in immune disorders of the skin, such as fibroblasts, keratinocytes and melanocytes, but especially Langerhans cells. The strength of the immune response can be determined for example through the amount of cytokines produced (such as interferon-gamma), detection of activation markers on dendritic cells (such as MHCII or CD86) or the number of activated CD8-positive T cells in the skin. Immunostimulants for the purpose of the present invention are, in particular, plant immunostimulants which are obtained, for example, from Echinacea pallida or Echinacea purpurea, cytokines such as, for example, interleukins, interferons and colony-stimulating factors, and bacterial constituents or molecules which mimic the latter [such as bacterial DNA and unmethylated oligodeoxynucleotides with CpG sequences, and constituents of the bacterial cell wall or coat, especially the lipopolysaccharides and molecules derived therefrom, such as monophosphoryl-lipid A, muramyldipeptide (N-acetylmuramyl-L-alanyl-D-isoglutamine), and/or PamCys3, and other molecules such as tetanus toxoid, poly-L-arginine or MHCII peptides].

The term "antibiotics" encompasses, for example, penicillins, cephalosporins, tetracyclines, aminoglycosides, macrolide antibiotics, lincosamides, gyrase inhibitors, sulfonamides, trimethoprim, polypeptide antibiotics, nitroimidazole derivatives, amphenicol, in particular actinomycin, alamethicin, alexidine, 6-aminopenicillanic acid, amoxicillin, amphotericin, ampicillin, anisomycin, antiamoebin, antimycin, aphidicolin, azidamfenicol, azidocillin, bacitracin, beclomethasone, benzathine, benzylpenicillin, bleomycin, bleomycin sulfate, calcium ionophore A23187, capreomycin, carbenicillin, cefacetrile, cefaclor, cefamandole nafate, cefazolin, cefalexin, cefaloglycin, cefaloridine, cefalotin, cefapirin, cefazolin, cefoperazone, ceftriaxone, cefuroxime, cephalexin, cephaloglycin, cephalothin, cephapirin, cerulenin, chloramphenicol, chlortetracycline, chloramphenicol diacetate, ciclacillin, clindamycin, chlormadinone acetate, chlorpheniramine, chromomycin A3, cinnarizine, ciprofloxacin, clotrimazole, cloxacillin, colistine methanesulfonate, cycloserine, deacetylanisomycin, demeclocycline, 4,4'-diaminodiphenyl sulfone, diaveridine, dicloxacillin, dihydrostreptomycin, dipyridamole, doxorubicin, doxycycline, epicillin, erythromycin, erythromycin stolate, erythromycin ethyl succinate, erythromycin stearate, ethambutol, flucloxacillin, fluocinolone acetonide, 5-fluorocytosine, filipin, formycin, fumaramidomycin, furaltadone, fusidic acid, geneticin, gentamycin, gentamycin sulfate, gliotoxin, gramicidin, griseofulvin, helvolic acid, hemolysin, hetacillin, kasugamycin, kanamycin (A), lasalocid, lincomycin, magnesidin, melphalan, metacycline, meticillin, mevinolin, micamycin, mithramycin, mithramycin A, mithramycin complex, mitomycin, minocycline, mycophenolic acid, myxothiazole, natamycin, nafcillin, neomycin, neomycin sulfate, 5-nitro-2-furaldehyde semicarbazone, novobiocin, nystatin, oleandomycin, oleandomycin phosphate, oxacihin, oxytetracycline, paromomycin, penicillin, pecilocin, pheneticillin, phenoxymethylpenicillin, phenyl aminosalicylate, phleomycin, pivampicillin, polymyxin B, propicillin, puromycin, puromycin aminonucleoside, puromycin aminonucleoside 5'-monophosphate, pyridinol carbamate, rolitetracycline, rifampicin, rifamycin B, rifamycin SV, spectinomycin, spiramycin, streptomycin, streptomycin sulfate, sulfabenzamide, sulfadimethoxine, sulfamethizole, sulfamethoxazole, tetracycline, thiamphenicol, tobramycin, troleandomycin, tunicamycin, tunicamycin A1 homolog, tunicamycin A2 homolog, valinomycin, vancomycin, vinomycin A1, virginiamycin M1, viomycin and/or xylostasin.

The term "antiinfectives" encompasses, for example, antimycotics, agents with antiparasitic effect and virustatics, in particular amphotericin, vifonazole, buclosamide, quinoline sulfate, chlormidazole, chlorphenesin, chlorquinaldol, clodantoin, cloxiquine, cyclopirox olamine, dequalinium chloride, dimazole, fenticlor, flucytosine, griseofulvin, ketoconazole, miconazole, natamycin, sulbentine, tioconazole, toinaftate, antiretroviral agents and/or herpes remedies.

The term "antiallergics" encompasses, for example, substances from the class of globulins, corticoids or antihistamines, in particular beclomethasone and derivatives thereof, betamethasone cortisone and derivatives thereof, dexamethasone and derivatives thereof, bamipine acetate, buclizine, clemastine, clemizole, cromoglicic acid, cyproheptadine, diflucortolone valerate, dimetotiazine, diphenhydramine, diphenylpyraline, ephedrine, fluocinolone, histapyrrodine, isothipendyl, methdilazine, oxomemazine, paramethasone, prednylidene, theophylline, and/or tolpropamine tritoqualine.

The term "mitogens", "chemokines" and "cytokines" encompass, for example, interferon-alpha, interferon-beta, interferon-gamma, interleukin-1, interleukin-2, interleukin-7, interleukin-10, interleukin-12, interleukin-18, GM-CSF, MIP-1-alpha/beta, RANTES, EGF, basic or acidic FGF, PDGF, IGF, VEGF, TGF-beta and/or TNF-alpha.

The term "dermatics" encompasses, for example, shale oil sulfonates, tar and tar derivatives, astringents, antihidrotics, acne remedies, antipsoriatics, antiseborrheic agents and/or enzyme preparations for the treatment of skin defects.

Consequently, a further aspect of the invention is the use of the polypeptide of the invention or a polynucleotide, a vector or a cell or a composition of the invention for the production of a medicament for the therapy of a proliferative disease, an immune disease, in particular an autoimmune disease, an infectious disease, a vascular disease, rheumatoid disease, in particular osteoarthritis and rheumatoid arthritis or a disease in which cells in or adjacent a disease site express and in particular overexpress, if compared with healthy or normal tissue, α_{V}β₃ and/or α_{V}β₅ integrin, and inflammatory diseases.

Since the expression or over expression of α_{V}β₃ and/or α_{V}β₅ integrins has been reported in particular for proliferative diseases this type of diseases are preferred diseases which can be treated with a polypeptide, a polynucleotide, a vector, a cell or a composition of the present invention. Particular preferred proliferative diseases are selected from the group consisting of carcinomas of the gastrointestinal or colorectal tract, liver, pancreas, kidney, bladder, prostate, endometrium, ovary, testes, melanoma, dysplastic oral mucosa, invasive oral cancers, small cell and non-small cell lung carcinomas, hormone-dependent breast cancers, independent breast cancers, transitional and squamous cell cancers, neurological malignancies including neuroblastoma, gliomas, astrocytomas, osteosarcomas, soft tissue sarcomas, hemangioamas, endocrinological tumors, hematologic neoplasias including leukemias, lymphomas, and other myeloproliferative and lymphoproliferative diseases, carcinomas in situ, hyperplastic lesions, adenomas, fibromas, histiocytosis, chronic inflammatory proliferative diseases, vascular proliferative diseases and virus-induced proliferative diseases.

Furthermore the polypeptides and composition of the present invention can be used or diagnostic purposes in particular, if they are attached to a marker as defined above. Therefore, another aspect of the present inventions is the use of a polypeptide or composition of the present invention for the diagnosis of a disease and in particular of diseases selected from the group of proliferative diseases, immune diseases, in particular autoimmune diseases, infectious diseases, vascular diseases, rheumatoid diseases, inflammatory diseases and diseases associated with an increase or decrease of the expression of α_{V}β₃ and/or α_{V}β₅ integrin. The detection of the marker *in vitro* in, for example, a biopsy of a patient or *in vivo* in a patient by, for example, tomographic methodologies will allow the detection of sites of neovascularization within a specimen or within a patient.

The following examples are included to demonstrate preferred embodiments of the invention. It should be appreciated by those of skill in the art that the techniques disclosed in the examples that follow represent techniques discovered by the inventors to function well in the practice of the invention, and thus can be considered preferred modes for its practice. However, those of skill in the art should, in light of the present disclosure, appreciate that many changes can be made in the specific embodiments that are disclosed without departing from the spirit and scope of the invention as set out in the appended claims. All references cited are incorporated herein by reference.

### Description of the Figures

- **Fig. 1:**: Binding of carboxyfluorescein-labelled RGD-10 to HUVECs. Thin line - cells without peptide, bold line - cells incubated with CF-labelled RGD-10.
- **Fig. 2:**: Titration of inhibition of RGD-10 binding by peptides RGD-10 or RGD-4C analyzed by cell ELISA.
- **Fig. 3:**: Structure of lipopeptides consisting of a peptide ligand with an acetylated Nterminus, the doo spacer linked to a dipalmitoyl-glycero-succinyl-lysine anchor.
- **Fig. 4:**: A) Binding of RGD10 liposomes to HUVEC or B16.F10. Liposomes containing the RGD10-LP or RGD10-LP3 lipopeptide at a conc. of 0.1 mol% (thin line), 1 mol% (normal line), or 5 mol% (bold line) were analyzed for binding to HUVECs or B16.F10 (marked with 0). Binding of liposomes lacking lipopeptides is indicated as dotted line. B.) Liposomes containing the RGD10LP3 lipopeptide at a conc. of 1 mol% were analyzed for binding to MeWo or A431 cells. Dotted line = cells alone, normal line = liposomes without lipopeptide, bold line, RGD10-LP3 liposomes.
- **Fig. 5:**: Competition experiments. Co-incubation of RGD10-LP3 liposomes (1 mol% lipopeptide) with 100 µM synthetic peptides RGD10 (A) or RGD-4C (B) completely inhibited binding of liposomes to HUVEC. In contrast, an RGE peptide (C) or a molecule consisting of 5 doo units did not cause any inhibition. Thin line = cells alone, normal line = plus peptide or 5 doo, bold line = liposomes without competitor.
- **Fig. 6:**: Cell binding and internalization of RGD-10-LP3 liposomes. Fluorescence microscopy analysis of binding of RGD10-LP3 liposomes or liposomes lacking lipopeptide (untargeted liposomes) to HUVECs at 4°C or 37°C. While at 4°C a cell surface staining is observed for RGD10-LP3 liposomes, incubation at 37°C for 2 hrs resulted in a perinuclear accumulation of fluorescence indicative for internalization of the liposomes.
- **Fig. 7:**: Pharmacokinetic analysis of liposomes containing 1 mol% LP1, 1 mol% LP3, or 0.1 mol% LP3.
- **Fig. 8:**: Antitumor effects of LP3-liposomes (Dox-LP3 liposomes) loaded with doxorubicin in comparison to untargeted liposomes (Dox liposomes) or free doxorubicin analyzed in a C26 murine colon carcinoma tumor model.

### Examples

### Example 1: Selection of novel RGD peptides

Two composed peptide libraries (CPL4b, CPL4c) containing a RGD motif flanked by cysteine residues were generated. Six amino acid positions, three preceding and three following the central -CRGDC- motif were randomized introducing the codon BNK (B = G,T,C; K = G,T; N = A,C,G,T) encoding all amino acids except Lys, Ile, Met, Thr, and Asn. Lysine residues were avoided in order to facilitate a directional chemical coupling through the N-terminal amino group. CPL4b differed from CPL4c by an Asp-Gly sequence at the C-terminus. Peptides were selected on human dermal microvascular endothelial cells (HDMEC), a human melanoma cell line (MeWo), or a mixture of murine melanoma cell lines B16.F1 and B16.F10.

A total of 15 different sequences, with some sequences occurring several times were identified, which are summarized in Table I.

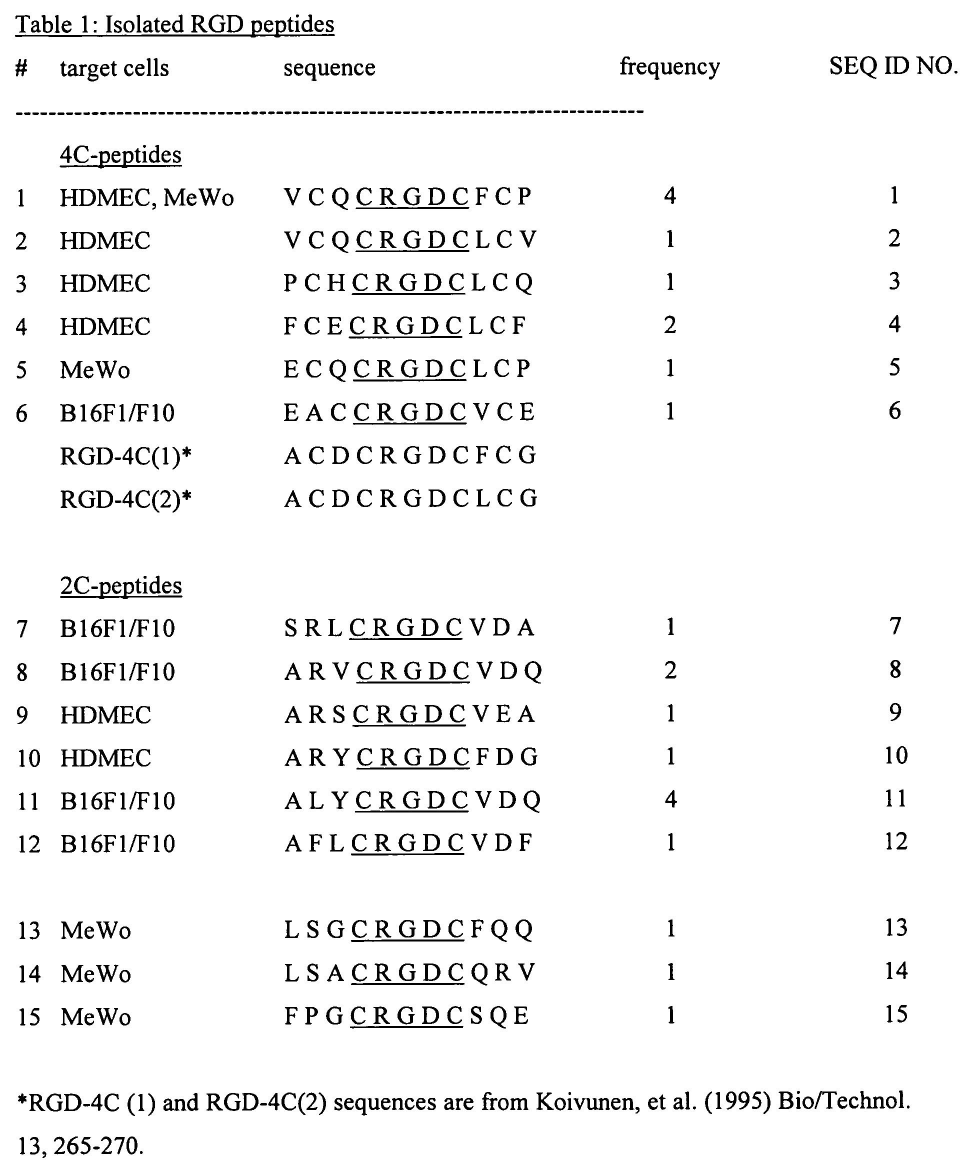

Except for two peptides selected on MeWo, all peptides were from the CPL4b library. The identified sequences can be divided into two groups. Peptides of group one (4C-RGD peptides) contain four cysteine residues either at positions 4, 6, 10, and 12 (peptides 1-5) or at positions 5, 6, 10 and 12 (peptide 6), with positions 6 and 10 corresponding to the fixed cysteine residues. Peptides 1-5 all contained a hydrophilic residue at position 5 (Q, E, H) and a hydrophobic residue at position 11 (L, F). Peptides of group two (2C-RGD peptides) contain only the two fixed cysteine residues at position 6 and 10. 4 out 6 peptides have an arginine residue at position 4 and an aspartate or glutamate residue at position 12 (peptides 7-12). These peptides preferred an alanine residue at position 3 and a valine residue at position 11.

### Example 2: Characterization of peptide RGD-10

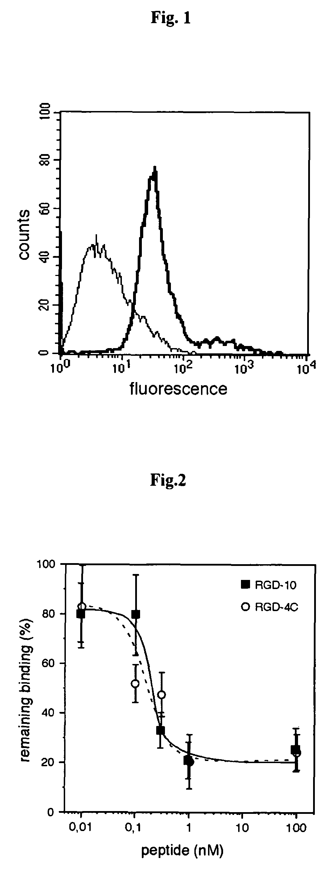

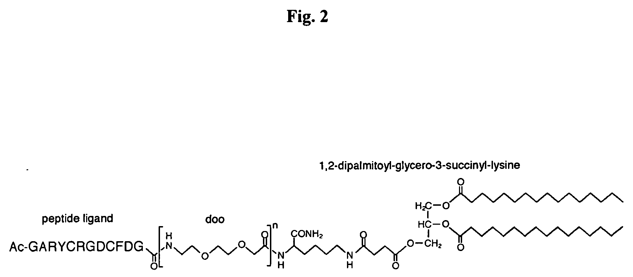

Analysis of 4C and 2C peptides for binding to endothelial or melanoma cells showed binding activities similar to or better then RGD-4C known to be a high affinity ligand of αᵥ-integrins and included as control. For further analysis we selected 2C-RGD peptide RGD-10 (DGARYCRGDCFDG). The RGD-10 peptide was synthesized with the sequence GARYCRGDCFDG, thus lacking the C-terminal aspartate residue and resulting in a neutral net charge. Direct binding of RGD-10 to HUVEC was demonstrated with a synthetic carboxyfluorescein-labeled RGD-10 peptide (Fig. 1). RGD-10 peptide bound with comparable strength as RGD-4C to HDMEC. For these experiments RGD-4C was oxidized in a two-step process to obtain disulfide bonds between C1-C4 and C2-C3 described to be essential for high affinity binding (Assa-Munt, *et al*. (2001) Biochemistry 40: 2373-2378). Compared to RGD-4C, RGD-10 contains only 2 cysteine residues, which facilitates synthesis of oxidized peptides using a one-step oxidation reaction. Furthermore, RGD-10 contains two aromatic residues, which allows spectrophotometrical detection at 280 nm. Competition experiments with free RGD-10, RGD-4C, and a non-RGD control peptide showed that both RGD peptides bind to the same epitope as RGD-4C. A titration of competition gave identical IC₅₀ values of approximately 0.2 µmol/l for RGD-10 and RGD-4C (Fig. 2). Thus, RGD-10 binds with the same affinity to integrin-expressing cells as RGD-4C, however, is much simpler to produce.

### Example 3: Binding of RGD10-lipopeptide containing liposomes to endothelial and melanoma cells

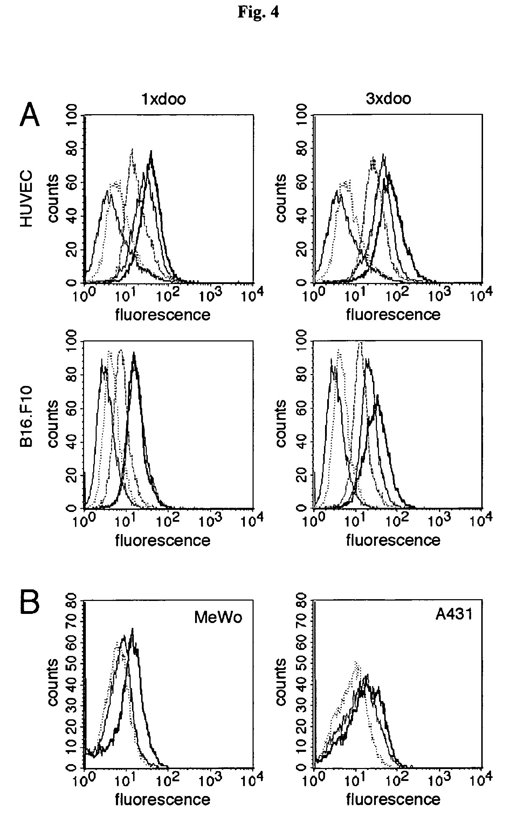

A novel lipopeptide structure composed of a 1,2-dipalmitoyl-glycero-3-succinyl-lysine anchor to which the RGD-10 peptide was coupled using 1 or 3 copies of 8-amino-3,6-dioxaoctanoic acid (doo) as spacer (Fig. 3) was designed.

Liposomes had the following lipid composition: 35 mol% cholesterol, 32,1 mol% palmitoyl-oleoyl-phosphatidylcholine (POPC), 14,7 mol% dilauroyl-phosphatidylethanolamine (DLPE), 18.2 mol% milk derived SM. The lipopeptideanchors were added at a molar ratio of 0.1 to 3 mol% reducing the concentration of lipids proportionally. All lipids were purchased from Avanti Polar Lipids (USA) and lipopeptides were synthesized by emc (Germany). Lipids and lipopeptides were used without further purification. Liposomes were prepared from dried lipid films by rehydration. For this purpose lipids and lipopeptides were dissolved in chloroform or chloroform/methanol (1:1) and mixed at the indicated ratios. For binding studies 0,03 mol% rhodamine-DPPE was added. Lipids were dried using a rotary evaporator and residual solvent was removed under high vacuum. Lipid films were then rehydrated with PBS pH 7.4 to a final lipid concentration of 10 µmol/ml. Liposomes were extruded 21-times through 50 nm membranes. All lipopeptide-containing liposomes had an average size of 76 +/- 2 nm. Thus the incorporation of 1 mol% lipopeptide corresponds to a theoretical value of approximately 350 surface-displayed ligands per liposome. Tumor cells were incubated with liposomes (50 nmol lipid) at 4°C for 30 min and subsequently analyzed by flow cytometry for cell binding activity.

With liposomes containing these RGD-10 lipopeptides (RGD10-LP1 with a 1-doo spacer, RGD10-LP3 with a 3-doo spacer) specific binding to endothelial cells as well as melanoma cells could be detected. Strong binding was observed for human umbilical vein endothelial cells (HUVEC) and to some weaker extent for murine melanoma cell line B16.F10 (Fig. 4A). Analysis of mean fluorescence revealed a 2-3 fold stronger binding to HUVECs compared to B16.F10. Only weak binding was seen with MeWo (Fig. 4B). No binding was observed with A431 tumor cells included as integrin-negative cell line (Fig. 4B). Binding experiments with liposomes containing 0.1, 1, or 5 mol% RGD10-LP1 or RGD10-LP3 showed that binding to these cells increased with increasing concentrations of lipopeptides (Fig. 4A). A 1.5 to 2-fold stronger binding was observed for 1 mol% LP1 or LP3 compared to 0.1 mol% lipopeptide. In addition, liposomes containing RGD10-LP3 showed increased binding (1.5 to 2-fold) to HUVEC or B16.F10 compared to liposomes containing RGD10-LP1, indicating that the longer doo-spacer improves binding to the cell surface.

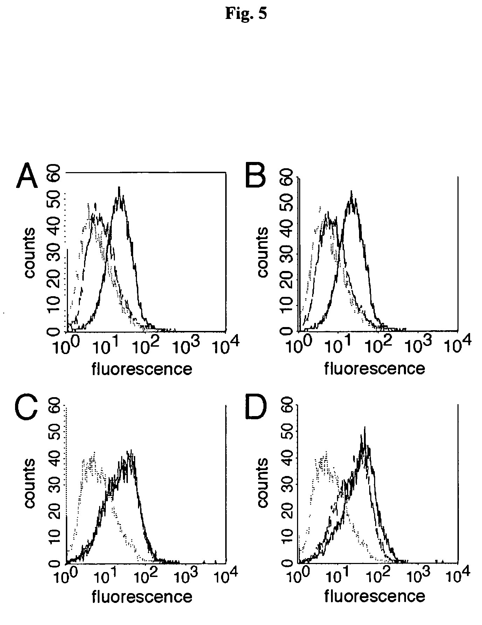

In order to demonstrate that binding is mediated by the RGD-10 peptide, various competition experiments were performed. Synthetic peptides RGD-10 or RGD-4C but not an irrelevant control peptide completely inhibited binding of RGD10 LP1-containing liposomes to HUVEC or B16.F10 (Fig. 5). In addition, no inhibition of binding was observed with a RGE peptide further demonstrating specificity of the RGD-10 ligand. Thus, binding to integrin-expressing cells is specifically mediated by the RGD peptide. Incubation of RGD10-liposomes with excess amount of a molecule consisting of 5 doo units (5-doo) did not inhibit binding to HUVECs indicating that the spacer region of the lipopeptide consisting of 1 or 3 doo units does not contribute to cell binding.

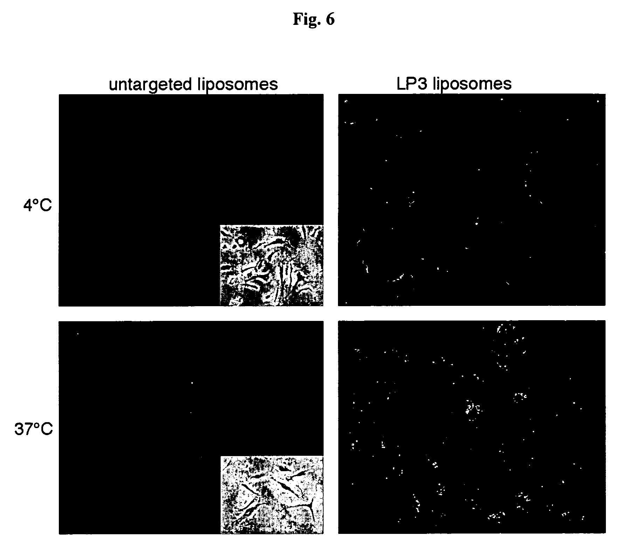

Fluorescence microscopy of HUVECs incubated with RGD10-LP3 liposomes at 37°C for 1 hr revealed a perinuclear staining pattern indicative for internalized liposomes. In contrast, only cell surface staining was observed after incubation at 4°C (Fig. 6). In these experiments, no staining was observed with liposomes lacking the RGD10 lipopeptide. These experiments demonstrate that RGD-targeted liposomes are taken up by integrin-expressing cells.

### Example 4: Pharmacokinetics of RGD10-LP3 liposomes

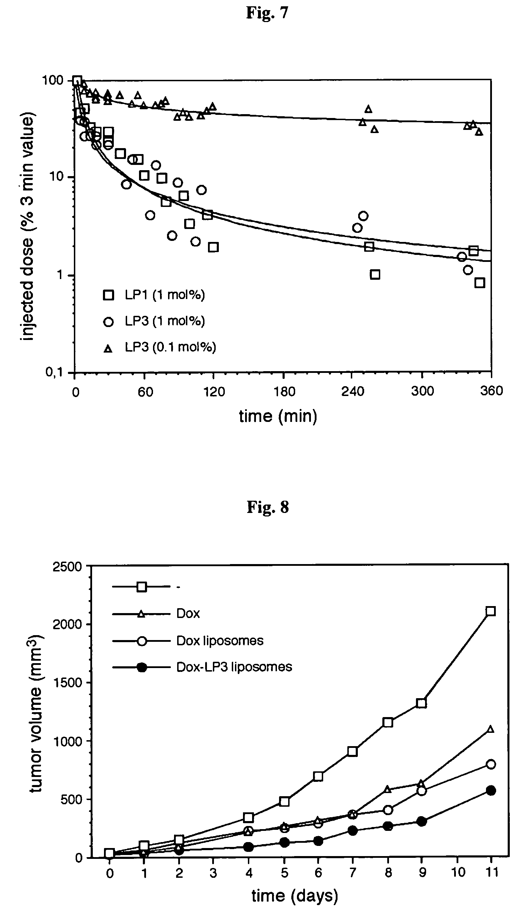

Pharmacokinetics was analyzed in nude mice injected with ³H-cholesteryloleylether-labelled liposomes containing different amounts of lipopeptide (Fig. 7). Liposomes were prepared as described in example 3. Liposomes containing 1 mol% RGD10-LP1 or RGD10-LP3 were rapidly eliminated from the blood stream with a t_{1/2} of approximately 9-10 minutes. Reduction of the amount of lipopeptide incorporated into liposomes to 0.1 mol% prolonged serum residence time to 102 min as calculated for the 50% value (t_{1/2}α = 44 min, t_{1/2}β = 9.2 h). This value is close to the serum residence time of liposomes lacking lipopeptides, which is approximately 108 min. Measuring organ distribution 6 hours postinjection we found for all lipopeptide-containing formulations accumulation in the liver (51-54% injected dose) and spleen (4-11% injected dose) and only marginal accumulation in the kidneys and lung.

### Example 6: Antitumor effects of doxorubicin-loaded RGD10 liposomes

Based on the pharmacokinetic data we used RGD10-LP3 liposomes containing 0.1% lipopeptide for analysis of in vivo antitumor effects. For this purpose liposomes were loaded with doxorubicin and analyzed in a C26 colon carcinoma mouse model.

Doxorubicin was encapsulated into liposomes (see example 3 for composition) containing 0.1% lipopeptide. As control we included liposomes lacking RGD-10 lipopeptide. Lipid films were rehydrated with 300 mmol/l citrat buffer pH 4.0. After extrusion the pH of the external aqueous solution was adjusted to pH 7.4 with NaOH. Liposomes were heated to 60°C and doxorubicin in PBS was added and incubated for 15 min. A drug to lipid ratio of 1:5 (w/w) was used in these experiments. Unencapsulated doxorubicin was removed by ultrafiltration using Vivaspin columns (centrifugation at 2800 rpm for 10 min). Encapsulation efficiency was determined by HPLC analysis of doxorubicin on a RP 18 column and was found to be in the range of 70-90%.

Pharmacodynamic experiments were performed in a C26 murine colon carcinoma model. For this purpose tumor cells were injected subcutaneously into nude mice. Tumor-bearing mice were then treated with doxorubicin-loaded liposomes injecting a doxorubicin dose of 4 mg/kg body weight at days 1, 3 and 6 after tumors had reached a size of approximately 50-100 mm³.

These experiments revealed an improved antitumor effect of doxorubicin-loaded RGD10-LP3 liposomes compared to free doxorubicin and to untargeted liposomes (Fig. 8). After 11 days tumor growth was inhibited by 45% (calculated from the tumor volume) compared to mice treated with untargeted liposomes. This improved antitumor effect was also reflected by a prolonged mean survival time, which was 18 days for animals treated with doxorubicin-loaded RGD10-LP3 liposomes, compared to 15 days for untargeted liposomes.

## Claims

1. A polypeptide comprising at least one binding peptide having an amino acid sequence as indicated by the general formula (I) X₁X₂X₃Y₁Y₂Y₃Y₄Y₁X₄X₅X₆ or by the general formula (II) X₇X₈X₉Y₁Y₂Y₃Y₄Y₁X₁₀Y₁X₁₁, wherein
Y₁ is Cys
Y₂ is Arg
Y₃ is Gly
Y₄ is Asp
X₁ is Ala, Leu, Phe or Ser, in particular Ala or Leu;
X₂ is Arg, Leu, Phe, Pro, or Ser, in particular Arg or Ser;
X₃ is Ala, Gly, Leu, Ser, Tyr, or Val, in particular Gly, Leu, or Tyr;
X₄ is Gln, Phe, Ser, or Val, in particular, Phe or Val;
X₅ is Arg, Asp, Glu, or Gln, in particular Asp or Gln;
X₆ is Ala, Gln, Glu, Gly, Phe, or Val, in particular Ala, Gln or Gly;
X₇ is Glu, Phe, Pro, or Val, in particular Glu or Val;
X₈ is Ala or Cys, in particular Cys;
X₉ is Asp, Cys, Gln, or His, in particular Gln;
X₁₀ is Leu, Phe, or Val, in particular Leu; and
X₁₁ Gln, Phe, Pro, or Val, in particular Pro,
wherein X₁, X₂, X₃, X₄, X₅, X₆, X₇, X₈, X₉, X₁₀, X₁₁, Y₁, Y₂, Y₃, Y4 are independently of each other the D or L amino acid or the amino acid residue mimetic of the respectively indicated amino acid;
or said amino acid sequence, which lacks 1 or 2, preferably 1, amino acid(s) from the N-terminus or C-terminus or 1 amino acid from the N- and C-terminus.

2. The polypeptide of claim 1, wherein:
X₁ is Ala or Leu;
X₂ is Arg or Ser;
X₃ is Gly, Leu, or Tyr
X₄ is Phe or Val;
X₅ is Asp or Gln;
X₆ is Ala, Gln, or Gly;
X₇ is Glu or Val;
X₈ is Cys;
X₉ is Gln;
X₁₀ is Leu; and
X₁₁ is Pro.

3. The polypeptide of claim 1, wherein the amino acid sequence is as shown in SEQ ID NOs 1 to 15.

4. The polypeptide of one of claims 1 to 3 having a length of 100, preferably 20 and most preferably 12 amino acids.

5. The polypeptide of one of claims 1 to 4, which comprises at least one amino acid sequence selected from the group consisting of a cytokine, a chemokine, a growth factor, an adhesion molecule, an antibody light and/or heavy chain, a single chain antibody, a toxin, an enzyme, a receptor ligand, a lytic peptide, a membrane insertion sequence and a fluorescent protein or fragments thereof.

6. The polypeptide of one of claims 1 to 5, which is attached to at least one chemical moiety.

7. The polypeptide of claim 6, wherein the chemical moiety is selected from the group consisting of a spacer, a marker, a tag, a lipid, in particular a phospholipid, a drug, a capping group and a spacer attached to a second chemical moiety.

8. The polypeptide of claim 7, wherein the spacer is selected from the group consisting of bifunctional polyethylenglycol and derivatives thereof, oligopeptides comprising between 1 to 40 natural or synthetic amino acids, 8-amino-3,6-dioxatanoic acid (doo), and (doo)ₙ, with n = 2-10.

9. The polypeptide of claim 7, wherein the marker is selected from the group consisting of an electron dense molecule, a paramagnetic molecule, a superparamagnetic molecule, a radioactive molecule, a non-radioactive isotope, and a fluorescent molecule.

10. The polypeptide of claim 7, wherein the lipid is selected from the group consisting of glycerides, glycerophospholipides, glycerophosphinolipids, glycerophosphonolipids, sulfolipids, sphingolipids, phospholipids, isoprenolides, steroids, stearines, steroles, and carbohydrate containing lipids.

11. The polypeptide of claim 10, wherein the phospholipid is selected from the group consisting of phosphatidylcholine (PC), phosphatidylserine (PS), and phosphatidylethanolamine (PE), in particular distearoylphosphatidyl (DSPE) or alpha-(dipalmitoylphosphatidyl (DPP).

12. The polypeptide of claim 7, wherein the lipid is selected from the group consisting of N-caproylamine-PE, N-dodecanylamine-PE, phophatidylthioethanol, N-[4-(p-maleimidomethyl)cyclohexane-carboxamide-PE (N-MCC-PE), N-[4-(p-maleimidophenyl)butyramide]-PE (N-MPB), N-[3-(2-pyridyldithio)propionate]-PE (N-PDP), N-succinyl-PE, N-glutaryl-PE, N-dodecanyl-PE, N-biotinyl-PE, N-biotinyl-cap-PE, phosphatidyl-(ehtylene glycol), PE-polyethylene glycol (PEG)-carboxylic acid, PE-PEG-maleimide, PE-PEG-PDP, PE-PEG-amine, PE-PEG-biotin, PE-PEG-HNS, dipalmitoyl-glycerosuccinyl-lysine, alpha-methoxy-omega-(1,2-dioctadecenoyloxy glyceryl) (DO), alpa-methoxy-omega-(1,2-ditetradecenoyloxy glyceryl) (DT).

13. The polypeptide of claim 7, wherein the second chemical moiety is selected from the group consisting of a drug, a marker, a tag, and a lipid.

14. A polynucleotide encoding at least one polypeptide of claims 1 to 5.

15. The polynucleotide of claim 14 which is DNA or RNA.

16. A vector containing the polynucleotide of claim 14 or 15.

17. The vector of claim 10, wherein the polynucleotide is operatively linked to expression control sequences allowing expression in prokaryotic and/or eukaryotic host cells.

18. A host cell genetically engineered with the polynucleotide of claim 14 or 15 or the vector of claim 16 or 17.

19. A transgenic non-human animal containing a polynucleotide of claim 14 or 15, a vector of claim 16 or 17 and/or a host cell of claim 18.

20. An antibody specifically binding to the amino acid sequence within the polypeptides of claims 1 to 13.

21. A composition comprising at least one polypeptide of one of claims 1 to 13 and at least one further component selected from the group consisting of liposomes, virosomes, microspheres, niosomes, dentrimers, stabilizers, buffers, excipients and additives.

22. The composition of claim 21, wherein the polypeptide is integrated into or attached to the liposome, microspheres, niosomes, dentrimers, or virosome.

23. The composition of claim 21 or 22, wherein the liposome or virosome comprises lipids selected from the group consisting of glycerides, glycerophospholipides, glycerophosphinolipids, glycerophosphonolipids, sulfolipids, sphingolipids, phospholipids, isoprenolides, steroids, stearines, steroles, and carbohydrate containing lipids.

24. The composition of one of claims 21 to 23, wherein the liposome or virosome comprises cholesterol (CH) and sphingomyelin (SM).

25. The composition of claim 24, wherein CH and SM are present in relation to the total molar lipid composition of the liposome or virosome at a molar ratio of 40 to 60 mol% and 10 to 20 mol%, respectively.

26. The composition of claim 25, wherein CH and SM are present in relation to the total molar lipid composition of the liposome or virosome at a molar ratio of 48 to 52 mol% and 12 to 16 mol%, respectively.

27. The composition of one of claims 24 to 26 further comprising PE and/or PC.

28. The composition of claim 27, wherein PE and PC are present in relation to the total molar lipid composition of the liposome or virosome at a molar ratio of 5 to 25 mol% and 15 to 40 mol%, respectively.

29. The composition of claims 21 to 28 further comprising a drug selected from the group consisting of analgesics, antirheumatics, anthelminthics, antiallergics, antianemics, antiarrhythmics, antibiotics, angiogenesis inhibitors, antiinfectives, antidemenics (nootropics), antidiabetics, antidotes, antiemetics, antivertiginosics, antiepileptics, antihemorrhagics, antihypertonics, antihypotonics, anticoagulants, antimycotics, antitussiv agents, antiviral agents, beta-receptor and calcium channel antagonists, broncholytic and antiastmatic agents, chemokines, cytokines, mitogens, cytostatics, cytotoxic agents and prodrugs thereof, dermatics, hypnotics and sedatives, immunosuppressants, immunostimulants, peptide or protein drugs, in particular hormones and physiological or pharmacological inhibitors of mitogens, chemokines, or cytokines or their respective prodrugs. Of course it is also envisioned that a liposome of the invention comprises more than one drug at once.

30. The compositions of claim 29, wherein the cytostatics and cytotoxic drugs are selected from the group consisting of alkylating substances, anti-metabolites, antibiotics, epothilones, nuclear receptor agonists and antagonists, anti-androgenes, anti-estrogens, platinum compounds, hormones and antihormones, interferons and inhibitors of cell cycle-dependent protein kinases (CDKs), inhibitors of cyclooxygenases and/or lipoxygenases, biogeneic fatty acids and fatty acid derivatives, including prostanoids and leukotrienes, inhibitors of protein kinases, inhibitors of protein phosphatases, inhibitors of lipid kinases, platinum coordination complexes, ethyleneimenes, methylmelamines, trazines, vinca alkaloids, pyrimidine analogs, purine analogs, alkylsulfonates, folic acid analogs, anthracendiones, substituted urea, methylhydrazin derivatives, in particular acediasulfone, aclarubicine, ambazone, aminoglutethimide, L-asparaginase, azathioprine, bleomycin, busulfan, calcium folinate, carboplatin, carpecitabine, carmustine, celecoxib, chlorambucil, cis-platin, cladribine, cyclophosphamide, cytarabine, dacarbazine, dactinomycin dapsone, daunorubicin, dibrompropamidine, diethylstilbestrole, docetaxel, doxorubicin, enediynes, epirubicin, epothilone B, epothilone D, estramucin phosphate, estrogen, ethinylestradiole, etoposide, flavopiridol, floxuridine, fludarabine, fluorouracil, fluoxymesterone, flutamide fosfestrol, furazolidone, gemcitabine, gonadotropin releasing hormone analog, hexamethylmelamine, hydroxycarbamide, hydroxymethylnitrofurantoin, hydroxyprogesteronecaproat, hydroxyurea, idarubicin, idoxuridine, ifosfamide, interferon α, irinotecan, leuprolide, lomustine, lurtotecan, mafenide sulfate olamide, mechlorethamine, medroxyprogesterone acetate, megastrolacetate, melphalan, mepacrine, mercaptopurine, methotrexate, metronidazole, mitomycin C, mitopodozide, mitotane, mitoxantrone, mithramycin, nalidixic acid, nifuratel, nifuroxazide, nifuralazine, nifurtimox, nimustine, ninorazole, nitrofurantoin, nitrogen mustards, oleomucin, oxolinic acid, pentamidine, pentostatin, phenazopyridine, phthalylsulfathiazole, pipobroman, prednimustine, prednisone, preussin, procarbazine, pyrimethamine, raltitrexed, rapamycin, rofecoxib, rosiglitazone, salazosulfapyridine, scriflavinium chloride, semustine streptozocine, sulfacarbamide, sulfacetamide, sulfachlopyridazine, sulfadiazine, sulfadicramide, sulfadimethoxine, sulfaethidole, sulfafurazole, sulfaguanidine, sulfaguanole, sulfamethizole, sulfamethoxazole, co-trimoxazole, sulfamethoxydiazine, sulfamethoxypyridazine, sulfamoxole, sulfanilamide, sulfaperin, sulfaphenazole, sulfathiazole, sulfisomidine, staurosporin, tamoxifen, taxol, teniposide, tertiposide, testolactone, testosteronpropionate, thioguanine, thiotepa, tinidazole, topotecan, triaziquone, treosulfan, trimethoprim, trofosfamide, UCN-01, vinblastine, vincristine, vindesine, vinblastine, vinorelbine, and zorubicin, or their respective derivatives or analogs thereof.

31. Use of a polypeptide of one of claims 1 to 13 or of a composition of one of claims 21 to 30 for the production of a medicament for the therapy of proliferative diseases, immune diseases, in particular autoimmune diseases, infectious disease, a vascular diseases, rheumatoid diseasse, in particular osteoarthritis and rheumatoid arthritis or diseases in which cells in or adjacent a disease site express α_{V}β₃ and/or α_{V}β₅ integrin, and inflammatory diseases.

32. The use of claim 31, wherein the proliferative disease is selected from the group consisting of carcinomas of the gastrointestinal or colorectal tract, liver, pancreas, kidney, bladder, prostate, endometrium, ovary, testes, melanoma, dysplastic oral mucosa, invasive oral cancers, small cell and non-small cell lung carcinomas, hormone-dependent breast cancers, independent breast cancers, transitional and squamous cell cancers, neurological malignancies including neuroblastoma, gliomas, astrocytomas, osteosarcomas, soft tissue sarcomas, hemangioamas, endocrinological tumors, hematologic neoplasias including leukemias, lymphomas, and other myeloproliferative and lymphoproliferative diseases, carcinomas in situ, hyperplastic lesions, adenomas, fibromas, histiocytosis, chronic inflammatory proliferative diseases, vascular proliferative diseases and virus-induced proliferative diseases.

33. The use of a polypeptide of claims 1 to 13 and/or a composition of claims 21 to 30 for the diagnosis of proliferative diseases, immune diseases, infectious diseases, vascular diseases, rheumatoid diseases, inflammatory diseases, and diseases associated with an increase or decrease of the expression of αᵥβ₃ and/or αᵥβ₅ integrin.
